# FASCICULE DE BREVET EUROPEEN

(11) **EP 3 092 589 B1**
(45) Date de publication et mention de la délivrance du brevet: **08.11.2023**
(21) Numéro de dépôt: 15701558.7
(22) Date de dépôt: 09.01.2015
(51) Int. Cl.: G16B 45/00, G16B 50/30, G06T 7/33, G06T 7/90

(54) **PROCEDE DE TRAITEMENT DE DONNEES D'IMAGERIE MOLECULAIRE ET SERVEUR DE DONNEES CORRESPONDANT**
VERFAHREN ZUR VERARBEITUNG BILDGEBUNGSDATEN UND ZUGEHÖRIGER DATENSERVER
METHOD FOR PROCESSING MOLECULAR IMAGING DATA AND CORRESPONDING DATA SERVER

(30) Priorité: 10.01.2014 FR 1450176
(43) Date de publication de la demande: 16.11.2016
(73) Titulaire: Aliri France, 59120 Loos (FR)
(72) Inventeur: PAMELARD, Fabien, F-59280 Armantieres (FR); STAUBER, Jonathan M., F-59320 Haubourdin (FR)
(74) Mandataire: Cabinet Becker et Associés
(86) Numéro de dépôt international: PCT/FR2015/050051
(87) Numéro de publication internationale: WO 2015/104512

(56) Documents cités:
- WO-A1-2009/156897
- IVO KLINKERT ET AL: "Methods for full resolution data exploration and visualization for large 2D and 3D mass spectrometry imaging datasets", INTERNATIONAL JOURNAL OF MASS SPECTROMETRY, vol. 362, 24 December 2013 (2013-12-24), pages 40-47, XP055477103, NL ISSN: 1387-3806, DOI: 10.1016/j.ijms.2013.12.012
- Suits Frank ET AL: "Correlation Queries for Mass Spectrometry Imaging", Analytical Chemistry, vol. 85, no. 9, 7 May 2013 (2013-05-07), pages 4398-4404, XP055829220, US ISSN: 0003-2700, DOI: 10.1021/ac303658t Retrieved from the Internet: URL:https://pubs.acs.org/doi/pdf/10.1021/a c303658t>
- RÜBEL OLIVER ET AL: "OpenMSI: A High-Performance Web-Based Platform for Mass Spectrometry Imaging", ANALYTICAL CHEMISTRY, vol. 85, no. 21, 5 November 2013 (2013-11-05), pages 10354-10361, XP055829223, US ISSN: 0003-2700, DOI: 10.1021/ac402540a Retrieved from the Internet: URL:https://pubs.acs.org/doi/pdf/10.1021/a c402540a>
- NORRIS ET AL: "Processing MALDI mass spectra to improve mass spectral direct tissue analysis", INTERNATIONAL JOURNAL OF MASS SPECTROMETRY, ELSEVIER SCIENCE PUBLISHERS , AMSTERDAM, NL, vol. 260, no. 2-3, 4 January 2007 (2007-01-04), pages 212-221, XP005827570, ISSN: 1387-3806

## Description

### DOMAINE TECHNIQUE DE L'INVENTION

La présente invention porte sur un procédé de traitement de données d'imagerie moléculaire basé sur l'utilisation d'une structure de base de données à haute volumétrie, ainsi que sur le serveur de données correspondant. L'imagerie moléculaire englobe de façon générale toutes les techniques d'imagerie permettant essentiellement d'observer le fonctionnement moléculaire des organes et/ou organismes ex vivo ou in vivo par des moyens les moins invasifs possibles ou perturbants le moins possible les systèmes biologiques ou biophysiques observés.

### ARRIERE PLAN TECHNOLOGIQUE

L'imagerie moléculaire connaît un essor considérable dans l'évolution du nombre de technologies notamment en imagerie par spectrométrie de masse (MSI pour "Mass Spectrometry Imaging" en anglais), imagerie par résonnance magnétique (MRI pour "Magnetic Résonance Imaging"), ou encore la spectrométrie Raman. L'utilisation de ces technologies permet de réaliser des études de bio-distribution de composés ciblés endogènes ou exogènes tels que les pesticides, les médicaments, les protéines ou les lipides afin d'étudier leurs rôles dans des systèmes biologiques. Les applications de ces technologies avec le concours de logiciels permettent également de rechercher de potentiels bio-marqueurs de manière non supervisée.

Toutefois, l'augmentation des données à analyser et à interpréter en imagerie moléculaire constitue une limitation pour les analyses synthétiques sur plusieurs jeux de données associés chacun à une image. Les données deviennent alors difficilement exploitables pour des représentations synthétiques ou analysables de façon statistique.

En effet, la haute volumétrie des informations des jeux de données est souvent supérieure à la taille de la mémoire vive des ordinateurs classiques (par opposition aux supers calculateurs). En imagerie par spectrométrie de masse par exemple, des images de 50 000 positions ne permettent plus de stocker l'intégralité de l'information brute en mémoire et nécessitent donc la réalisation de calculs pour réduire l'impact en mémoire et permettre l'exploitation de ces images. Les calculs réduisent les données et par conséquent induisent des biais en ne prenant pas en compte toute l'information disponible. De plus, les outils d'analyse actuels ne présentent aucun moyen de normalisation de plusieurs jeux de données entre eux.

Il existe de nombreux formats de stockage liés principalement aux constructeurs des automates d'acquisition des jeux de données. Toutefois, aucun de ces formats n'est adapté et optimisé pour être interrogé de façon fine et rapide. Certains domaines de l'imagerie moléculaire ont mis au point des formats de stockage tels que l'Analyse 7.5 que l'on retrouve en IRM (Imagerie par Résonance Magnétique) et en imagerie par spectrométrie de masse. Dans l'imagerie par spectrométrie de masse, on trouve également le format iMZML qui ne constitue toutefois pas un système de stockage pour manipuler des données d'imagerie.

Des formats du type HDF5 (pour "Hierarchical Data Format 5" en anglais) basés sur une organisation hiérarchique des données ont récemment été utilisés dans le stockage des données à haute volumétrie en imagerie par spectrométrie de masse. Ces formats permettent de construire des interfaces d'interrogation à distance via des navigateurs Internet. Ils permettent des calculs statistiques plus rapides qu'avec des fichiers non hiérarchisés. Ces formats ne permettent toutefois pas d'obtenir une analyse comparative globale et interrogeable des données dans des études de plusieurs jeux de données simultanément.

Il existe donc le besoin d'un procédé permettant d'analyser, de comparer, et d'interroger plusieurs jeux de données les uns par rapport aux autres sans perte d'information afin d'éviter d'introduire des biais d'analyse.

### OBJET DE L'INVENTION

La présente invention vise à répondre efficacement à ce besoin en proposant un procédé de traitement par ordinateur de données d'imagerie moléculaire comme défini dans la revendication 1.

Plus généralement, l'invention propose de traiter des jeux de données, acquis avantageusement de manière automatisée, en découpant en tronçons les spectres moléculaires associés à chacune des positions spatiales des ensembles de positions spatiales composant lesdits jeux de données. A chaque spectre correspond ainsi une succession de tronçons de spectre, comprenant à eux tous l'ensemble des informations moléculaires du spectre associé. Selon l'invention, l'étape de découpage en tronçon est réalisée de manière automatisée, pendant l'acquisition des données spectrales ou pendant l'analyse des données spectrales.

Dans un premier mode de réalisation les tronçons successifs ne se chevauchent pas et sont simplement adjacent, de sorte qu'aucune information moléculaire d'un spectre considéré n'est perdue ni redondante. Dans un autre mode de réalisation, certains des tronçons d'un spectre considéré peuvent se chevaucher partiellement, de sorte que certaines informations moléculaires peuvent être contenues dans deux tronçons de spectre successifs d'un même spectre.

L'invention permet ainsi de réduire de manière substantielle la durée de chargement des données à analyser, dans la mesure où il est possible de récupérer, par sélection de tronçons, l'information utile dans la base de données. Il est également possible de tronçonner les données spectrales dès l'enregistrement des données, pendant leur acquisition, de sorte que les tronçons de données spectrales sont intégrés directement pendant l'analyse de l'échantillon par un procédé d'imagerie moléculaire.

Du fait du découpage en tronçons du signal, sans réduction du spectre, l'invention permet en outre de travailler sans réduction de l'information dans le cadre d'études combinées de pharmacocinétique, de pharmacodynamique et de quantification de molécules endogènes ou exogènes dans une même analyse afin d'observer dans son ensemble des événements physiologiques comme des méthodes de screening (comparaison de distribution de plusieurs candidats médicaments), méthodes de quantification, méthodes d'étude d'occupation de récepteurs ou de criblage tissulaire, d'étude de métabolisme ou de reconstruction 3D. L'invention permet également d'optimiser l'utilisation de la mémoire nécessaire aux traitements statistiques et aux représentations des données d'imagerie moléculaire sur plusieurs jeux de données.

Selon une mise en oeuvre, le procédé de traitement ou d'enregistrement de données de l'invention comporte l'étape de visualiser l'information moléculaire d'intérêt sous forme d'un ensemble de cartographies de données correspondant chacune à un jeu de données.

Avantageusement, les spectres moléculaires sont découpés selon un pas déterminé, qui peut être identique ou différent d'un spectre à un autre.

Selon une mise en oeuvre, le procédé de traitement ou d'enregistrement de données de l'invention comporte les étapes suivantes:
- découper l'ensemble d'informations moléculaires en tronçons de spectre suivant au moins un pas prédéterminé,
- insérer un axe de référence établissant une correspondance entre des index de points des différents tronçons de spectre et l'information moléculaire correspondante, et
- sélectionner le tronçon de spectre contenant l'information moléculaire d'intérêt en fonction de l'axe de référence et du pas prédéterminé.

Selon une mise en oeuvre, avant ou après l'insertion des tronçons dans la base de données, ledit procédé comporte au moins une étape de pré-traitement consistant en un alignement spectral et/ou une soustraction de bruit de fond et/ou une normalisation intra jeu de données.

Selon une mise en oeuvre, le procédé de traitement ou d'enregistrement de données de l'invention comporte l'étape de redimensionner les positions spatiales des jeux de données ayant des tailles spatiales différentes de manière à s'aligner sur le jeu de données ayant la taille spatiale la plus fine.

Selon une mise en oeuvre, le procédé de traitement ou d'enregistrement de données de l'invention comporte en outre l'étape d'extraire un ensemble de données, telles qu'une intensité maximum, une intensité moyenne, une aire sous un pic, un rapport signal sur bruit de chaque pic d'intérêt de l'ensemble d'informations moléculaires.

Selon une mise en oeuvre, l'extraction est effectuée en fonction de critères de sélection de pic définis par un critère de qualité d'un rapport signal sur bruit et/ou une résolution spectrale.

Selon une mise en oeuvre, le procédé de traitement ou d'enregistrement de données de l'invention comporte l'étape de normaliser les ensembles d'informations moléculaires de la pluralité des jeux de données entre eux de manière à pouvoir comparer les différents jeux de données entre eux.

Selon une mise en oeuvre, pour réaliser l'étape de normalisation, ledit procédé peut comporter les étapes suivantes:
- choisir une molécule ou plusieurs molécules endogènes ou exogènes de référence communes à l'ensemble des jeux de données,
- calculer, pour chaque jeu de données, un facteur de normalisation en fonction de cette ou ces molécules de référence, et
- corriger l'information moléculaire d'intérêt avec ce facteur de normalisation pour obtenir un ensemble de jeux de données normalisés.

Selon une mise en oeuvre, le facteur de normalisation est défini à partir d'au moins une molécule de référence présente sur un ensemble d'échantillons correspondant aux jeux de données ou dans une zone particulière de référence des échantillons correspondant aux jeux de données.

Selon une mise ne oeuvre, le procédé de traitement ou d'enregistrement de données de l'invention comporte l'étape de visualiser, comparer, et analyser, sous forme d'une pluralité de cartographies de données issues chacune d'un jeu de données normalisé, l'information moléculaire d'intérêt avec une échelle de couleur commune à l'ensemble des cartographies.

Selon une mise en oeuvre, l'échelle de couleur commune est basée sur une intensité la plus petite et la plus grande de tous les jeux de données pour la molécule d'intérêt. Selon une mise en oeuvre, lesdits jeux de données sont obtenus par un procédé de spectrométrie de masse.

Selon une mise en oeuvre, lesdits jeux de données sont obtenus par un procédé d'imagerie de type Tomographie par Emission de Positons (TEP) ou d'imagerie par Résonance Magnétique (IRM).

L'invention a également pour objet un serveur de données comme défini dans la revendication 14.

### BREVE DESCRIPTION DES FIGURES

L'invention sera mieux comprise à la lecture de la description qui suit et à l'examen des figures qui l'accompagnent. Ces figures ne sont données qu'à titre illustratif mais nullement limitatif de l'invention.
La figure 1 représente de manière schématique les différentes étapes d'un procédé de spectroscopie de masse pour l'obtention de jeux de données exploités par le procédé selon la présente invention;
La figure 2 est un schéma synoptique du système intégré de gestion de base de données permettant la mise en oeuvre du procédé de traitement de données selon la présente invention;
La figure 3 est une représentation schématique de l'étape permettant d'obtenir les tronçons des spectres associés à chaque position d'un jeu de données ;
La figure 4 représente les étapes permettant d'établir la correspondance entre une information moléculaire et le tronçon de spectre correspondant ;
La figure 5 montre de manière schématique les étapes de normalisation inter-images permettant de comparer de manière précise plusieurs images entre elles.

Les éléments identiques, similaires, ou analogues conservent les mêmes références d'une figure à l'autre.

### DESCRIPTION D'UN EXEMPLE DE REALISATION DE L'INVENTION

L'exemple de mise en oeuvre du procédé selon l'invention qui suit est décrit pour une méthode de spectrométrie de masse de type MALDI. Bien entendu, on pourrait de manière sensiblement identique utiliser un autre dispositif d'imagerie que le MALDI, comme par exemple les sources : SIMS, DESI, LAESI, DIOS, ICP, Microscope MALDI, SNOM, SMALDI, LA-ICP, ESI (extraction liquide sur tissu), MILDI, JEDI, ELDI etc. Le procédé est également applicable à toute autre méthode permettant de produire des données exploitables par une méthode d'imagerie moléculaire comme par exemple la TEP ("Tomographie par Emission de Positons") ou l'IRM (Imagerie par Résonance Magnétique).

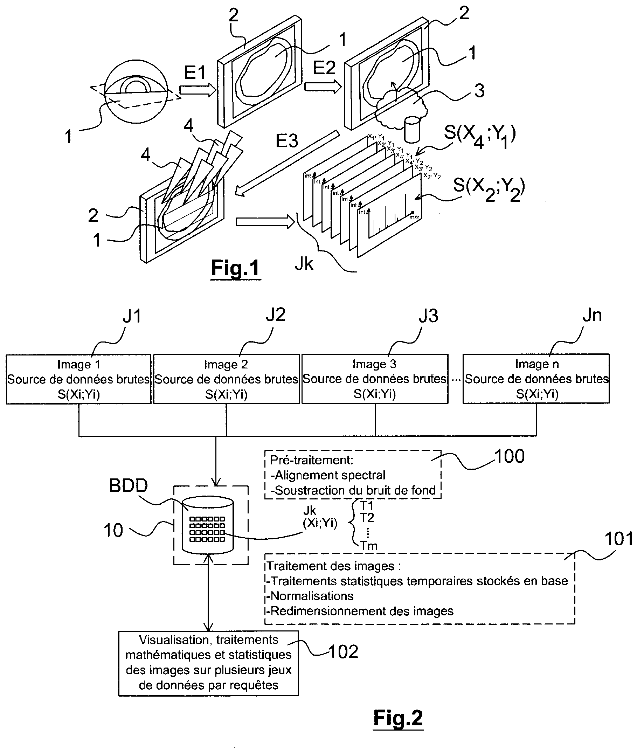

La figure 1 montre de manière schématique les différentes étapes mises en oeuvre par un procédé de spectrométrie de masse MALDI. Une première étape E1 consiste à découper, en général par cryo-section, une tranche de tissu 1 qui est ensuite posée sur une lame 2. On dépose ensuite dans une étape E2 une fine couche uniforme de matrice ionisante 3 sur la tranche de tissu 1. L'étape d'acquisition E3 consiste à générer des impulsions laser automatisées 4 ayant une taille prédéfinie, par exemple de l'ordre de 100 micromètres par zone d'acquisition, afin d'ioniser les molécules de la matrice 3. Les molécules ainsi ionisées sont analysées de façon connue en soi par un spectromètre de masse. On pourra se référer au document FR2973112 pour plus de détails sur la mise en oeuvre du procédé.

On obtient alors un jeu de données Jk défini par un ensemble de positions spatiales (Xi, Yj) à chacune desquelles est associé un ensemble d'informations moléculaires. Les positions spatiales sont définies en fonction d'un référentiel de référence d'axes X et Y. L'ensemble d'informations moléculaires est formé en l'occurrence notamment par un spectre S à deux dimensions indiquant une intensité en fonction d'une masse moléculaire. Par exemple, le jeu de données Jk obtenu présente 50000 intensités (soit 50000 points) par spectre sur 20000 positions spatiales Xi, Yj. Le procédé selon l'invention est basé sur le traitement d'une pluralité de jeux de données J1-Jn.

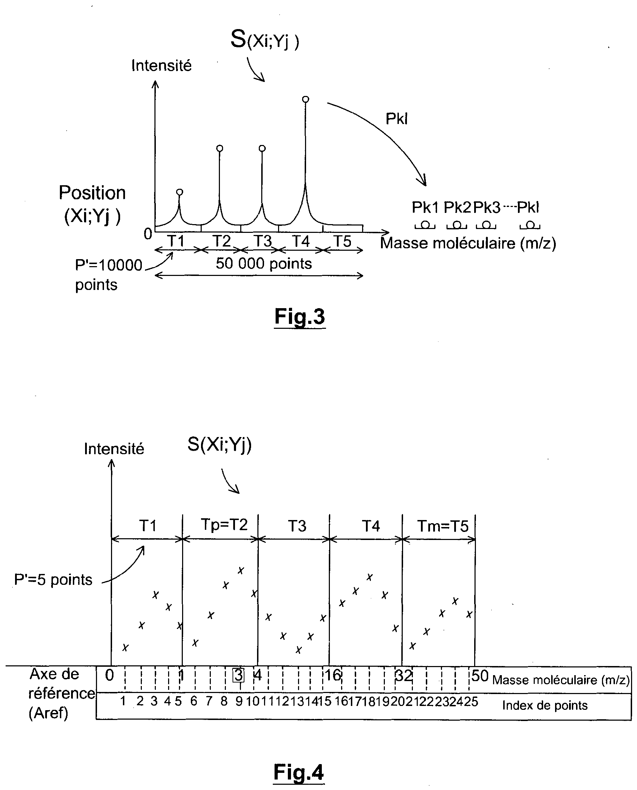

Comme cela est montré sur la figure 3, pour chaque jeu de données Jk susceptible d'être représenté sous forme d'une image, le spectre moléculaire S(Xi, Yj) associé à chaque position (Xi, Yj) est découpé en plusieurs tronçons de spectres T1-Tm. Les tronçons T1-Tm sont découpés suivant un pas prédéterminé P'. Par exemple, pour chaque spectre de 50000 points par position spatiale, chaque spectre pourra être découpé suivant un pas P' de 10000 points. Autrement dit, le spectre S(Xi, Yj) est découpé en cinq tronçons T1-T5 de 10000 points chacun. En variante, le pas P' utilisé pourra être variable.

On définit par ailleurs un axe de référence Aref, montré sur la figure 4, établissant une correspondance entre des index de points des différents tronçons T1-Tm et la masse moléculaire correspondante.

De préférence, avant insertion dans une base de données BDD, le procédé met en oeuvre au moins une étape de pré-traitement des spectres S(Xi, Yj) via un module 100. Cette étape de pré-traitement pourra consister en un alignement spectral. A cet effet, on considère des pics de référence et on décale le spectre de manière à positionner les valeurs de pics correspondants sur ces pics de référence. Il sera également possible d'effectuer une soustraction du bruit de fond en recalant le spectre en fonction d'un écart entre la valeur minimale du spectre et une valeur de référence à laquelle devrait correspondre la valeur minimale du spectre.

En outre, différents types de normalisation du signal (normalisation interne) pourront être réalisés (par un standard interne, par utilisation du total des intensités...). Autrement dit, on réalise ici une normalisation intra jeu de données, c'est-à-dire une normalisation pour chaque jeu de données indépendamment des autres jeux de données, ce qui correspond à des étapes connues en soi de l'homme du métier. Cette normalisation intra jeu de données est effectuée en complément des étapes ultérieures de normalisation entre les jeux de données J1-Jn détaillées ci-après (normalisation inter jeu de données) qui permettront de comparer, de manière précise et sûre, les jeux de données J1-Jn entre eux.

Par ailleurs, le procédé comporte l'étape d'extraire un ensemble de données, telles qu'une intensité maximum Pk1-Pkl, une intensité moyenne, une aire sous un pic, un rapport signal sur bruit de chaque pic d'intérêt des spectres S(Xi, Yj). Il est ainsi possible d'extraire l'intensité maximum de chaque pic Pk1-Pkl du spectre S(Xi, Yj) suivant une méthode dite de "PeakPicking" en anglais. L'extraction est effectuée en fonction de critères de sélection de pics définis par un critère de qualité du rapport signal sur bruit et/ou la résolution spectrale.

Les tronçons T1-Tm obtenus pour chaque position (Xi, Yj) de chaque jeu de données J1-Jn ainsi que l'axe de référence Aref et les pics Pk1-PKl précédemment extraits sont insérés dans la base de données BDD comme cela est montré à la figure 2. Ainsi, à chaque position (Xi, Yj) de chaque jeu de données J1-Jn sont associés un ensemble de tronçons indexés T1-TM et un ensemble de pics Pkl-PKl et de données complémentaires précitées (intensités moyennes, aires de chaque pic).

La base de données BDD est une base de données à haute volumétrie du type NoSQL (pour "Not only Structured Query Langage" en anglais) ou SQL permettant la visualisation, la gestion, l'interprétation et l'analyse statistique des données insérées. Le système de gestion de base de données peut par exemple être choisi parmi les systèmes non exhaustifs suivants: Hypertable, Haddoop, Cassandra, MongoDB, PolyBase, Hadoop on Azure, Hive, Pig. Le système de base de données assure la cohérence, la fiabilité, et la pertinence des données contenues. Des techniques de réplications et de sauvegardes peuvent permettre de garantir la fiabilité de l'information.

On décrit ci-après les étapes permettant la sélection de l'information pertinente dans la base de données BDD. Suite à une requête relative à une information moléculaire d'intérêt, on sélectionne le ou les tronçons des positions des ensembles de jeu de données J1-Jn contenant l'information moléculaire demandée. A cette fin, pour chaque position Xi, Yj de chaque jeu de données J1-Jn, le tronçon Tp contenant l'information moléculaire demandée est sélectionné en fonction de l'axe de référence Aref et de l'information moléculaire demandée.

Ainsi, sur la figure 4, on a illustré une découpe d'un spectre en tronçon suivant un pas P' de cinq points pour faciliter la compréhension de cette étape. Comme cela est souvent le cas pour les spectres issus d'une méthode de spectrométrie de masse, l'échelle des masses moléculaires n'est pas linéaire. Dans le cas où l'utilisateur requiert des informations relatives à une molécule ayant par exemple une masse moléculaire de valeur 3 m/z, l'axe de référence Aref permet d'établir que l'index du point correspondant à cette valeur de masse moléculaire est le 9, en sorte que l'on peut en déduire à l'aide du pas P' que le tronçon Tp contenant l'intensité de la masse moléculaire d'intérêt est le deuxième tronçon T2.

Alternativement, on peut utiliser une table qui stocke les bornes minimum et maximum des masses moléculaires et l'indexation des points de chaque tronçon T1-Tm correspondant. Dans ce cas, pour une information moléculaire demandée, on sélectionne l'identifiant du tronçon T1-Tm en fonction de ces bornes minimum et maximum.

Au sein de chaque tronçon T1-Tm sélectionné pour chacun des points de l'ensemble des jeux de données J1-Jn, l'intensité correspondant de la masse moléculaire demandée est alors sélectionnée. Il devient alors possible de visualiser, sous forme d'un ensemble de cartographies de données C1-CN correspondant chacune à un jeu de données J1-Jn, la répartition de l'intensité de présence de la molécule d'intérêt sur les différentes cartographies C1-CN. On attribue une couleur en fonction de l'intensité de l'information moléculaire sur une échelle de couleur. Alternativement, ou de façon complémentaire, une extraction des pics Pk1-PKl permet de produire rapidement des images fournissant une première analyse de la répartition de l'intensité d'une molécule d'intérêt.

De préférence, le procédé comporte l'étape de normaliser les informations moléculaires des jeux de données J1-Jn afin de pouvoir comparer plusieurs jeux de données entre eux (cf. module 101). A cet effet, on choisit une molécule de référence Mref commune à tous les jeux de données J1-Jn. Alternativement, on choisit plusieurs molécules endogènes ou exogènes communes à l'ensemble des jeux de données J1-Jn. Il est possible de tenir compte de la présence de la ou des molécules de référence Mref dans les échantillons complets ou plus spécifiquement dans une zone de référence des échantillons correspondants.

Plus précisément, pour chaque jeu de données J1-Jn, un facteur de normalisation Fnor est calculé en fonction de cette ou ces molécules de référence Mref. Les informations moléculaires d'intérêt sont alors corrigées avec ce facteur de normalisation Fnor de manière à obtenir un ensemble de jeux de données J1-Jn normalisés.

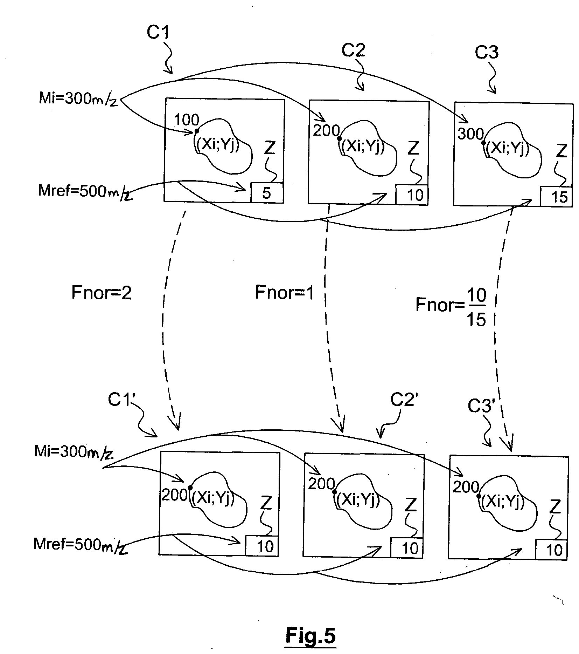

Par exemple, pour trois jeux de données correspondant aux cartographies C1-C3 représentées sur la figure 5, on choisit une molécule de référence Mref=500 m/z en tant que molécule normalisante (standard normalisant). Cette molécule Mref présente respectivement une valeur de 5, 10 et 15 dans la zone Z des cartographies C1-C3. Pour chaque cartographie C1-C3, on calcule un facteur de normalisation Fnor sur la base de chacune de ces valeurs de Mref :
Fnor= Moyenne des valeurs de Mref pour C1-C3 / valeur du Mref de la cartographie considérée. Dans l'exemple représenté à la figure 5, on obtient un facteur de normalisation Fnor respectivement de 2, 1, et 10/15 pour les cartographies C1, C2 et C3. Ce coefficient est alors appliqué à l'ensemble des valeurs d'intensité des spectres associés aux différentes positions de l'ensemble des jeux de données J1-Jn et en particulier aux valeurs d'intensité d'une molécule d'intérêt Mi=300 *m*/*z* à la position Xi; Yj. On obtient alors des cartographies normalisées C1'-C3', qui peuvent être directement comparées entre elles.

La normalisation permet ainsi de corriger les intensités liées à des changements de conditions expérimentales dus à la préparation de l'échantillon et à son analyse. En effet, dans l'exemple précédent, on remarque qu'il aurait été possible d'arriver à une conclusion erronée d'une présence plus forte de la molécule d'intérêt Mi=300 *m*/*z* dans l'échantillon correspondant à la cartographie C3, alors qu'après application du facteur de normalisation Fnor, l'étude fait ressortir une présence homogène de la molécule d'intérêt Mi dans les trois échantillons avec une intensité de 200 à la position Xi; Yj.

Suite à cette normalisation, il est alors possible de visualiser, comparer et analyser, sous forme de cartographies de données C1'-C3' normalisées l'information moléculaire avec une échelle de couleur commune à l'ensemble des cartographies C1'-C3' (cf. module 102). L'échelle obtenue est basée sur l'intensité la plus petite et la plus grande déterminées sur tous les jeux de données pour la molécule d'intérêt Mi.

Bien entendu, il est possible de produire plus de trois jeux de données et cartographies correspondantes pour analyser par regroupements statistiques plusieurs jeux de données J1-Jn de façon transverse sans avoir besoin de tous les charger en mémoire. On peut alors comparer les données d'imagerie pour en faire ressortir des marqueurs potentiels. Il est également possible de réaliser des regroupements de positions ayant un comportement commun afin de permettre une nouvelle interprétation des données.

Autrement dit, l'invention permet ainsi, dans un seul outil, de comparer et interroger les données pour l'obtention d'une information plus pertinente, normalisée et dont la qualité est quantifiable. Plusieurs jeux de données peuvent être chargés avec des contrôles qualités insérés dans chacune des images permettant ainsi de normaliser les données entre elles.

En outre, le procédé peut le cas échéant permettre de redimensionner les positions de jeux de données J1 et J2 du même échantillon biologique ayant des tailles spatiales différentes de manière à s'aligner sur le jeu de données ayant la plus fine taille spatiale. Il est alors possible de combiner le traitement d'une cartographie issue du spectromètre de masse (imagerie MALDI) avec le traitement d'une cartographie issue d'un système de type PET (Tomographie par Emission de Positons) ou IRM (Imagerie par Résonance Magnétique) ou IRM (Imagerie par Résonance Magnétique).

Il est également possible de représenter plusieurs jeux de données J1-Jn en codistribution dans une image unique. Il est alors possible de réaliser des requêtes du type "sélectionner toutes les valeurs v, w de toutes les positions x et y (de même taille spatiale) pour le champ z et a de la cartographie courante". Les champs z et a représentent les valeurs de deux technologies d'imagerie différente.

Il est également possible de réaliser des traitements statistiques temporaires qui pourront être ajoutés par des requêtes d'insertion dans la base de données BDD. Ainsi, le procédé selon l'invention apporte une grande flexibilité dans la mesure où il est possible d'ajouter de l'information sur chaque position de l'image à tout moment du procédé d'analyse.

Le procédé est basé sur une architecture client/serveur. La base de données BDD est interrogeable par une interface unique. La donnée est stockée et interrogée dans une architecture monoposte ou en cluster de serveurs accessible via en local ou via un réseau (interne ou externe). L'interface logicielle cliente peut être installée sur le poste de travail ou en version interface web. L'invention a également pour objet le serveur de données 10 comportant une mémoire stockant des instructions logicielles pour la mise en oeuvre d'au moins une partie des étapes du procédé de traitement d'une pluralité de jeux de données J1-Jn.

De plus, la quantité de données traitée peut croître facilement, en sorte que la volumétrie de la donnée à analyser n'est plus une limitation à l'analyse. L'insertion des données est performante quelle que soit la taille de la base de données BDD.

Bien entendu, la description qui précède a été donnée à titre d'exemple uniquement et ne limite pas le domaine de l'invention dont on ne sortirait pas en remplaçant les détails d'exécution par tous autres équivalents.

## Revendications

1. Procédé de traitement par ordinateur de données d'imagerie moléculaire comprenant une pluralité de jeux de données spectrales (J1-Jn), chaque jeu de données spectrales (J1-Jn) étant défini par un ensemble de positions spatiales (Xi, Yj) à chacune desquelles est associée un spectre moléculaire à au moins deux dimensions contenant un ensemble d'informations moléculaires (S(Xi, Yj)), **caractérisé en ce qu'**il comporte les étapes suivantes
- pour chaque jeu de données (J1-Jn), découper de manière automatisée le spectre moléculaire associé à chaque position (Xi, Yj) en plusieurs tronçons de spectre (T1-Tm) contenant chacun une partie des informations moléculaires dudit spectre, lesdits tronçons de spectre comprenant à eux tous l'ensemble des informations moléculaires du spectre moléculaire associé
- insérer les tronçons (T1-Tm) obtenus pour chaque position (Xi, Yj) de chaque jeu de données (J1-Jn) dans une base de données (BDD), en sorte qu'à chaque position (Xi, Yj) de chaque jeu de données (J1-Jn) est associé un ensemble de tronçons de spectre (T1-Tm) indexés,
- sélectionner dans la base de données (BDD), suite à une requête relative à une information moléculaire d'intérêt, le ou les tronçons (T1-Tm) contenant l'information moléculaire d'intérêt, et
- sélectionner, au sein de chaque tronçon (T1-Tm), ladite information moléculaire d'intérêt.

2. Procédé selon la revendication 1, **caractérisé en ce qu'**il comporte l'étape de visualiser l'information moléculaire d'intérêt sous forme d'un ensemble de cartographies de données (C1-Cn) correspondant chacune à un jeu de données (J1-Jn).

3. Procédé selon l'une quelconque des revendications 1 à 2, **caractérisé en ce qu'**il comporte les étapes suivantes:
- découper les spectres moléculaires (S(Xi, Yj)) en tronçons de spectre (T1-Tm) suivant au moins
un pas prédéterminé (P'),
- insérer un axe de référence (Aref) établissant une correspondance entre des index de points des différents tronçons (T1-Tm) et l'information moléculaire correspondante, et
- sélectionner le tronçon (T1-Tm) contenant l'information moléculaire d'intérêt en fonction de l'axe de référence (Aref) et du pas prédéterminé (P').

4. Procédé selon l'une quelconque des revendications 1 à 3, **caractérisé en ce que**, avant ou après l'insertion des tronçons (T1-Tm) dans la base de données (BDD), ledit procédé comporte au moins une étape de pré-traitement consistant en un alignement spectral et/ou une soustraction de bruit de fond et/ou une normalisation intra jeu de données (J1-Jn).

5. Procédé selon l'une quelconque des revendications 1 à 4, **caractérisé en ce qu'**il comporte l'étape de redimensionner les positions spatiales (Xi, Yj) des jeux de données (J1-Jn) ayant des tailles spatiales différentes de manière à s'aligner sur le jeu de données (J1-Jn) ayant la taille spatiale la plus fine.

6. Procédé selon l'une quelconque des revendications 1 à 5, **caractérisé en ce qu'**il comporte en outre l'étape d'extraire un ensemble de données, telles qu'une intensité maximum (Pk1-Pkl), une intensité moyenne, une aire sous un pic, un rapport signal sur bruit de chaque pic d'intérêt de l'ensemble des spectres moléculaires, et éventuellement **caractérisé en ce que** l'extraction est effectuée en fonction de critères de sélection de pic définis par un critère de qualité d'un rapport signal sur bruit et/ou une résolution spectrale.

7. Procédé selon l'une quelconque des revendications 1 à 6, **caractérisé en ce qu'**il comporte l'étape de normaliser les spectres moléculaires de la pluralité des jeux de données (J1-Jn) entre eux de manière à pouvoir comparer les différents jeux de données (J1-Jn) entre eux.

8. Procédé selon la revendication 7, **caractérisé en ce que** pour réaliser l'étape de normalisation, ledit procédé comporte les étapes suivantes:
- choisir une molécule ou plusieurs molécules endogènes ou exogènes de référence (Mref) communes à l'ensemble des jeux de données (J1-Jn),
- calculer, pour chaque jeu de données (J1-Jn), un facteur de normalisation (Fnor) en fonction de cette ou ces molécules de référence (Mref), et
- corriger l'information moléculaire d'intérêt avec ce facteur de normalisation pour obtenir un ensemble de jeux de données (J1-Jn) normalisés.

9. Procédé selon la revendication 8, **caractérisé en ce que** le facteur de normalisation (Fnor) est défini à partir d'au moins une molécule de référence (Mref) présente sur un ensemble d'échantillons correspondant aux jeux de données (J1-Jn) ou dans une zone particulière de référence (Z) des échantillons correspondant aux jeux de données (J1-Jn).

10. Procédé selon l'une quelconque des revendications 7 à 9, **caractérisé en ce qu'**il comporte l'étape de visualiser, comparer, et analyser, sous forme d'une pluralité de cartographies de données (C1'-Cn') issues chacune d'un jeu de données (J1-Jn) normalisé, l'information moléculaire d'intérêt avec une échelle de couleur commune à l'ensemble des cartographies.

11. Procédé selon la revendication 10, **caractérisé en ce que** l'échelle de couleur commune est basée sur une intensité la plus petite et la plus grande de tous les jeux de données (J1-Jn) pour la molécule d'intérêt.

12. Procédé selon l'une quelconque des revendications 1 à 11, **caractérisé en ce que** lesdits jeux de données (J1-Jn) sont obtenus par un procédé de spectrométrie de masse.

13. Procédé selon l'une quelconque des revendications 1 à 11, **caractérisé en ce que** lesdits jeux de données (J1-Jn) sont obtenus par un procédé d'imagerie de type Tomographie par Emission de Positons (TEP) ou d'Imagerie par Résonance Magnétique (IRM).

14. Serveur de données (10) comportant une mémoire stockant des instructions logicielles pour la mise en oeuvre des étapes du procédé de traitement d'une pluralité de jeux de données (J1-Jn) défini selon l'une quelconque des revendications précédentes.

## Patentansprüche

1. Verfahren zur rechnergestützten Verarbeitung molekularer Bildgebungsdaten, umfassend eine Vielzahl an Spektraldatensätzen (J1-Jn), wobei jeder Spektraldatensatz (J1-Jn) durch eine Reihe räumlicher Positionen (Xi, Yj) definiert ist, denen ein molekulares Spektrum mit wenigstens zwei Dimensionen zugeordnet ist, enthaltend eine Reihe molekularer Informationen (S(Xi, Yj), **dadurch gekennzeichnet, dass** es die folgenden Schritte umfasst
- automatisiertes Aufteilen, für jeden Datensatz (J1-Jn), des zu jeder Position (Xi, Yj) gehörenden molekularen Spektrums in mehrere Spektralabschnitte (T1-Tm), wobei jeder einen Teil der molekularen Informationen des Spektrums enthält, wobei die Spektralabschnitte zusammen die gesamten molekularen Informationen des dazu gehörenden molekularen Spektrums umfassen,
- Einfügen der Abschnitte (T1-Tm), die für jede Position (Xi, Yj) jedes Datensatzes (J1-Jn) erhalten wird, in eine Datenbank (BDD), so dass jede Position (Xi, Yj) jedes Datensatzes (J1-Jn) allen indizierten Spektralabschnitten (T1-Tm) zugeordnet wird,
- Auswählen des oder der Abschnitte (T1-Tm), enthaltend die molekulare Information von Interesse, in der Datenbank (BDD), nach einer Anfrage in Bezug auf eine molekulare Information von Interesse, und
- Auswählen der molekularen Information von Interesse in jedem Abschnitt (T1-Tm).

2. Verfahren gemäß Anspruch 1, **dadurch gekennzeichnet, dass** es den Schritt der Visualisierung der molekularen Information von Interesse in Form einer Reihe an Datenkarten (C1-Cn) umfasst, die jeweils einem Datensatz (J1-Jn) entsprechen.

3. Verfahren gemäß einem der Ansprüche 1 bis 2, **dadurch gekennzeichnet, dass** es die folgenden Schritte umfasst
- Aufteilen der molekularen Spektren (S(Xi, Yj)) in Spektralabschnitte (T1-Tm) nach wenigstens einem vorgegebenen Schritt (P'),
- Einfügen einer Referenzachse (Aref), die eine Entsprechung zwischen den Punktindizes verschiedener Abschnitte (T1-Tm) und der entsprechenden molekularen Information herstellt, und
- Auswählen des Abschnittes (T1-Tm), der die molekulare Information von Interesse enthält, in Abhängigkeit von der Referenzachse (Aref) und dem vorgegebenen Schritt (P').

4. Verfahren gemäß einem der Ansprüche 1 bis 3, **dadurch gekennzeichnet, dass** vor oder nach Einfügen der Abschnitte (T1-Tm) in die Datenbank (BDD) das Verfahren wenigstens einen Schritt der Vorbearbeitung umfasst, bestehend aus einem spektralen Alignment und/oder einer Subtraktion des Hintergrundrauschens und/oder einer intra Datensatz (J1-Jn) Normalisierung.

5. Verfahren gemäß einem der Ansprüche 1 bis 4, **dadurch gekennzeichnet, dass** es den Schritt der Größenänderung der räumlichen Positionen (Xi, Yj) der Datensätze (J1-Jn) mit unterschiedlichen räumlichen Größen umfasst, um sich an den Datensatz (J1-Jn) mit der kleinsten räumlichen Größe anzupassen.

6. Verfahren gemäß einem der Ansprüche 1 bis 5, **dadurch gekennzeichnet, dass** es außerdem den Schritt der Extraktion einer Reihe an Daten umfasst, wie zum Beispiel maximale Intensität (Pkl-Pk1), mittlere Intensität, eine Fläche unter einem Peak, Signal-Rausch-Verhältnis eines jeden Peaks von Interesse der gesamten molekularen Spektren, und gegebenenfalls **dadurch gekennzeichnet, dass** die Extraktion abhängig der Peak-Auswahlkriterien erfolgt, die durch ein qualitatives Kriterium eines Signal-Rausch-Verhältnisses und/oder einer spektralen Auflösung definiert sind.

7. Verfahren gemäß einem der Ansprüche 1 bis 6, **dadurch gekennzeichnet, dass** es den Schritt der Normalisierung der molekularen Spektren der Vielzahl an Datensätzen (J1-Jn) miteinander umfasst, um die verschiedenen Datensätze (J1-Jn) miteinander vergleichen zu können.

8. Verfahren gemäß Anspruch 7, **dadurch gekennzeichnet, dass** für die Durchführung des Normalisierungsschrittes das Verfahren die folgenden Schritte umfasst
- Auswählen eines oder mehrerer endogener oder exogener Referenz-Moleküle (Mref), die allen Datensätzen (J1-Jn) gemeinsam sind,
- Berechnen für jeden Datensatz (J1-Jn) eines Normalisierungsfaktors (Fnor) in Abhängigkeit dieses oder dieser Referenzmoleküle (Mref), und
- Korrigieren der molekularen Information von Interesse mit diesem Normalisierungsfaktor, um eine Reihe normalisierter Datensätze (J1-Jn) zu erhalten.

9. Verfahren gemäß Anspruch 8, **dadurch gekennzeichnet, dass** der Normalisierungsfaktor (Fnor) mithilfe wenigstens eines Referenzmoleküls definiert ist, das in einer Reihe von Proben, die den Datensätzen (J1-Jn) entsprechen, oder in einer bestimmten Referenzzone (Z) der Proben, die den Datensätzen (J1-Jn) entsprechen, vorhanden ist.

10. Verfahren gemäß einem der Ansprüche 7 bis 9, **dadurch gekennzeichnet, dass** es den Schritt der Visualisierung, des Vergleichs oder der Analyse in Form einer Vielzahl an Datenkarten (C1`-Cn'), die jeweils aus einem normalisierten Datensatz (J1-Jn) stammen, der molekularen Information von Interesse mit einer für alle Karten gemeinsamen Farbskala umfasst.

11. Verfahren gemäß Anspruch 10, **dadurch gekennzeichnet, dass** die gemeinsame Farbskala auf einer kleinsten und größten Intensität aller Datensätze (J1-Jn) für das Molekül von Interesse beruht.

12. Verfahren gemäß einem der Ansprüche 1 bis 11, **dadurch gekennzeichnet, dass** die Datensätze (J1-Jn) mit einem massenspektrometrischen Verfahren erhalten sind.

13. Verfahren gemäß einem der Ansprüche 1 bis 11, **dadurch gekennzeichnet, dass** die Datensätze (J1-Jn) mit einem bildgebenden Verfahren wie Positronenemissionstomographie (PET) oder Magnetresonanztomographie (MRT) erhalten sind.

14. Datenserver (10), umfassend einen Speicher zur Speicherung von Softwareanweisungen zur Durchführung der Schritte des Verfahrens zur Verarbeitung einer Vielzahl an Datensätzen (J1-Jn), das gemäß einem der vorhergehenden Ansprüche definiert ist.

## Claims

1. A process for computer processing molecular imaging data comprising a plurality of spectral datasets (J1-Jn), each spectral dataset (J1-Jn) being defined by a set of spatial positions (Xi, Yj) each of which is associated with a molecular spectrum in at least two dimensions containing a set of molecular information (S(Xi, Yj)), **characterized in that** it includes the following steps
- for each dataset (J1-Jn), automatically dividing the molecular spectrum associated with each position (Xi, Yj) into several spectrum sections (T1-Tm) each containing part of the molecular information of said spectrum, said spectrum sections comprising together the set of molecular information of the associated molecular spectrum
- inserting the sections (T1-Tm) obtained for each position (Xi, Yj) of each dataset (J1-Jn) in a database (BDD), so that each position (Xi, Yj) of each dataset (J1-Jn) is associated with a set of indexed spectrum sections (T1-Tm),
- selecting from the database (BDD), following a request relating to molecular information of interest, the section(s) (T1-Tm) containing the molecular information of interest, and
- selecting, within each section (T1-Tm), said molecular information of interest.

2. The process according to claim 1, **characterized in that** it includes the step of visualizing the molecular information of interest in the form of a set of data maps (C1-Cn) each corresponding to a dataset (J1-Jn).

3. The process according to any one of claims 1 to 2, **characterized in that** it includes the following steps:
- cutting the molecular spectra (S(Xi, Yj)) into spectrum sections (T1-Tm) according to at least one predetermined pitch (P'),
- inserting a reference axis (Aref) establishing a correspondence between point indexes of the different sections (T1-Tm) and the corresponding molecular information, and
- selecting the section (T1-Tm) containing the molecular information of interest according to the reference axis (Aref) and the predetermined pitch (P').

4. The process according to any one of claims 1 to 3, **characterized in that**, before or after the insertion of the sections (T1-Tm) into the database (BDD), said process includes at least one pre-processing step consisting of spectral alignment and/or background noise subtraction and/or intra-dataset normalization (J1-Jn).

5. The process according to any one of claims 1 to 4, **characterized in that** it includes the step of resizing the spatial positions (Xi, Yj) of the datasets (J1-Jn) having different spatial sizes so as to be aligned with the dataset (J1-Jn) having the finest spatial size.

6. The process according to any one of claims 1 to 5, **characterized in that** it further includes the step of extracting a set of data, such as a maximum intensity (Pk1-Pkl), an average intensity, an area under a peak, a signal-to-noise ratio of each peak of interest of all the molecular spectra, and optionally **characterized in that** the extraction is carried out according to peak selection criteria defined by a quality criterion of a signal-to-noise ratio and/or a spectral resolution.

7. The process according to any one of claims 1 to 6, **characterized in that** it includes the step of normalizing the molecular spectra of the plurality of datasets (J1-Jn) relative to each other so as to be able to compare the different datasets (J1-Jn) with each other.

8. The process according to claim 7, **characterized in that** to carry out the normalization step, said process includes the following steps:
- selecting a molecule or several endogenous or exogenous reference molecules (Mref) common to all the datasets (J1-Jn),
- calculating, for each dataset (J1-Jn), a normalization factor (Fnor) according to this or these reference molecules (Mref), and
- correcting the molecular information of interest with this normalization factor to obtain a set of normalized datasets (J1-Jn).

9. The process according to claim 8, **characterized in that** the normalization factor (Fnor) is defined from at least one reference molecule (Mref) present on a set of samples corresponding to the datasets (J1-Jn) or in a particular reference zone (Z) of the samples corresponding to the datasets (J1-Jn).

10. The process according to any one of claims 7 to 9, **characterized in that** it includes the step of visualizing, comparing, and analyzing, in the form of a plurality of data maps (Cl'-Cn') from each of a standardized dataset (J1-Jn), the molecular information of interest with a color scale common to all the maps.

11. The process according to claim 10, **characterized in that** the common color scale is based on a smallest and highest intensity of all the datasets (J1-Jn) for the molecule of interest.

12. The process according to any one of claims 1 to 11, **characterized in that** said datasets (J1-Jn) are obtained by a mass spectrometry process.

13. The process according to any one of claims 1 to 11, **characterized in that** said datasets (J1-Jn) are obtained by a process of imaging of Positron Emission Tomography (PET) type or of Magnetic Resonance Imaging (MRI).

14. A data server (10) including a memory storing software instructions for implementing the steps of the process for processing a plurality of datasets (J1-Jn) defined according to any one of the preceding claims.
